# EUROPEAN PATENT SPECIFICATION

(11) **EP 1 663 021 B1**
(45) Date of publication and mention of the grant of the patent: **17.06.2009**
(21) Application number: 04743598.7
(22) Date of filing: 28.07.2004
(51) Int. Cl.: A61B 17/17

(54) **A DRILL GUIDE ASSEMBLY**
BOHRLEHRE
ENSEMBLE GUIDE-FORET

(30) Priority: 22.09.2003 GB 0322084
(43) Date of publication of application: 07.06.2006
(73) Proprietor: Depuy International Limited, Beeston, Leeds LS11 8DT (GB)
(72) Inventor: MOORE, Gary, Wetherby, West Yorkshire LS22 7UE (GB); BIRKBECK, Alec, Leeds LS16 5AX (GB)
(74) Representative: Belcher, Simon James
(86) International application number: PCT/GB2004/003271
(87) International publication number: WO 2005/027755

(56) References cited:
- US-A- 4 896 663
- US-A- 6 156 069
- US-B2- 6 595 999

## Description

The present invention relates to a drill guide assembly for determining the axis for drilling a boré to receive a component of an orthopaedic joint prosthesis.

When preparing a bone to receive a component of a joint prosthesis, it can be important to establish the position of an axis which provides a reference against which the location of the prosthesis component is determined for implantation. The axis can conveniently be determined prior to any resection of the bone, relative to the natural bone tissue.

When the bone tissue provides the ball component of a ball and socket joint (for example the humeral component of a shoulder joint or, especially, the femoral component of a hip joint), the axis should be determined relative to its convex bearing surface: the prosthesis component should be implanted in alignment with that axis or at a predetermined orientation relative to the axis. It can be difficult for a surgeon to align a drill guide accurately relative to a bone prior to fixing the drill guide to the bone for use.

US-6595999 discloses a drilling jig which includes a drill guide tube with a rounded head which is supported in a housing. The head is able to pivot within the housing so that the axial orientation of the drill guide tube relative to the housing can be adjusted. The tube can be clamped against adjustment. The clamp comprises upper and lower housing parts which can be drawn together so as to grip the rounded head of the clamp on opposite sides thereof (see Figures 6 and 10). A transverse arm can be used to obtain high clamping forces. However, even using an arm, the forces by which the head is clamped in the housing can be insufficient to prevent movement of the drill guide when the jig is in use.

In one aspect the invention provides a drill guide assembly for determining the axis for drilling a bore to receive a component of an orthopaedic joint prosthesis, which comprises:
a. a drill guide which comprises a sleeve and a bulb at one end of the sleeve,
b. a frame which can be fastened to the bone, including a housing which defines a recess in which the drill guide bulb can be received with the drill guide sleeve extending out of the recess, in a direction away from the bone, so that the angular orientation of the drill guide sleeve relative to the housing can be adjusted by movement of the bulb within the recess, and
c. a clamp for locking the drill guide relative to the housing against angular adjustment,
in which the clamp comprises a lower pair of clamping surfaces provided by the drill guide bulb and the internal wall of the recess respectively, and an upper pair of clamping surfaces on the drill guide and the housing respectively, arranged so that the drill guide can be locked against angular adjustment by engagement between the frame clamping surfaces and the drill guide clamping surfaces of each of the lower and upper pairs, in which the upper clamping surface of the drill guide is spaced apart from the bulb along the drill guide sleeve.

The drill guide assembly of the invention has the advantage that the forces which have to be overcome if the drill guide is moved when the drill guide is clamped in the frame against angular movement can be arranged to be higher than when would be the case if the bulb is gripped on opposite sides by upper and lower housing parts. Secure locking of the drill guide is therefore facilitated.

The drill guide assembly of the invention allows the alignment of the drill guide sleeve relative to the bone axis to be finalised after fixing the assembly to the bone. The fixing of the assembly to the bone does not therefore have to be performed accurately because the final alignment of the drill guide sleeve is achieved after fixation, made possible by the translation of the drill guide sleeve relative to the frame when the locking mechanism is in its unlocked position.

Preferably, the frame upper clamping surface is provided on a collar portion which extends from the housing in a direction away from the bone, the collar portion being hollow so that the drill guide sleeve can extend through it. The collar will frequently provide a continuous surface which faces towards the corresponding clamping surface on the drill guide. Its side wall can be solid, or it can have openings extending through it. The collar can extend from the housing for the bulb and be formed with the housing as a single component, for example by casting or moulding. Alternatively, the collar can be formed as a separate component from the housing, and assembled with it, for example using cooperating screw threads.

Preferably, the frame upper clamping surface faces generally away from the patient's bone and the clamping surface of the recess faces generally towards the patient's bone. In this way, the side of the recess opposite to the lower frame clamping surface, which faces towards the bone, can be open. This can facilitate assembly of the frame. It can also facilitate cleaning. When the frame upper clamping surface can face towards the patient's bone, the clamping surface of the recess will then face away from the patient's bone.

Preferably, one of the upper clamping surfaces is provided by a washer, and the clamp includes an actuator which can act on the washer to urge it against the other of the upper clamping surfaces. When the washer provides the frame upper clamping surface, it can be provided on a collar which extends from the housing in a direction away from the bone. A washer can be provided on the drill guide sleeve, which can be urged against the frame upper clamping surface. A washer on the drill guide sleeve can be urged against the surface of a collar which extends from the housing and faces away from the housing.

Preferably, the assembly includes an actuator by which the frame clamping surfaces and the drill guide clamping surfaces of each of the lower and upper pairs can be forced together. Preferably, the actuator comprises a threaded nut. The threaded nut can act against a washer which provides one of the upper clamping surfaces, to force it towards the other of the upper clamping surfaces. For example, when the washer is provided on the drill guide sleeve, an actuator nut can be provided on an threaded external surface of the sleeve. It is an advantage of the assembly of the invention that adequate clamping forces can be applied through a threaded nut without having to use an elongate arm tool.

Preferably, at least one of the upper clamping surfaces is domed. It is particularly preferred that each of the upper clamping surfaces is domed; one of the surfaces can be convex and the other concave. This can facilitate angular adjustment of the drill guide within the frame.

Preferably, the assembly includes a resiliently deformable washer which is located between at least one of the upper clamping surfaces and the lower clamping surfaces. For example, an O-ring made from a resiliently deformable material (such as a silicone rubber) can be provided in a groove in one of the clamping surfaces. When one of the upper clamping surfaces is provided by a washer, an O-ring can be provided in a groove in the other of the upper clamping surfaces. The provision of a washer of a resiliently deformable material has the advantage that the tension that is applied to the pairs of clamping surfaces, to achieve satisfactory clamping, is less critical than if the clamp is provided by rigid clamping surfaces.

Preferably, the ratio of (a) the distance between the upper and lower clamping surfaces when the drill guide is clamped against angular adjustment to (b) the transverse dimension of the bulb measured perpendicular to the axis of the drill guide sleeve, is at least 1.3, more preferably at least about 1.7, for example at least about 2.0. A greater distance between the upper and lower clamping surfaces can mean that the forces which have to be overcome if the drill guide is moved when the drill guide is clamped in the frame against angular movement are higher than when the bulb is gripped on opposite sides by upper and lower housing parts.

Preferably, the surface of at least one of the bulb and the recess is rounded, at least over that part of the said surface (or surfaces) in which clamping takes place. For example, when the bulb has a rounded surface, this will generally be provided by a bulb which is defined by part of a sphere. For example, it can be approximately hemispherical. The other lower clamping surface can be rounded with a similar radius when surface-to-surface contact is required.

Preferably, the assembly of the invention allows for adjustment of the drill guide through an angle of arc of at least about 10°, more preferably at least about 20°, for example at least about 30°. The angle through which the orientation of the drill guide can be adjusted is dependent on the size of the opening through which the drill guide extends out of the recess in the housing, and also the internal dimension of any collar portion on the frame.

The drill guide sleeve preferably defines a bore in which a drill can be received. The size of the bore should be sufficient for the drill to be sliding fit, with minimum clearance which would allow play to reduce the accuracy of the location of the bore that is drilled in the bone. The sleeve can also be used to locate surgical instruments other than drill bits. Suitable drill guide sleeves might have bores with an internal transverse dimension of, for example, 2 to 5 mm.

Preferably, the frame provides a platform which defines a plane which is spaced apart from the bone and an axis of the assembly which extends perpendicular to the said plane, and in which the drill guide is mounted on the platform so that it can be translated relative to the frame generally in the plane of the platform. Translation of the drill guide sleeve in this way allows the location of a bore which is prepared by the drill to be changed, while maintaining the angular orientation relative to the bone axis. For example, the bore can be aligned with the axis, or located at a predetermined distance from the axis.

Preferably, the drill guide sleeve is mounted on the platform so that (a) it can be moved relative to the frame in the plane of the platform, and (b) its angular orientation relative to the plane of the platform can be adjusted, the locking mechanism being operable selectively to prevent one or both of (a) and (b).

Preferably, the assembly includes a lock for preventing translation of the drill guide relative to the frame, in which the clamp and the lock can be engaged and disengaged independently of one another.

The lock which prevents translation of the drill guide sleeve relative to the frame can comprise a pair of plates which cooperate with a frame plate (for example with provides the frame platform) located between them. Operation of the lock involves clamping the frame plate between the lock plates, for example by means of a threaded nut which acts on one of the lock plates.

Preferably, the assembly includes an alignment stylus connected to the drill guide sleeve to move with the drill guide sleeve relative to the frame, the stylus including a first limb which extends in a direction generally towards the bone, to facilitate assessment of the alignment of the drill guide sleeve relative to anatomical features of the bone. Preferably, the stylus can be moved rotatably around the drill guide sleeve. Preferably, the stylus can be moved relative to the frame independently of any movement of the drill guide sleeve relative to the frame. In particular, it is preferred that the stylus should be capable of being moved around the drill guide sleeve while the locking mechanism is engaged to prevent movement of the sleeve relative to the frame.

The stylus can include a second limb extending from the first limb in a direction generally towards the axis of the assembly, the second limb having a stylus tip. The second limb can be used to determine the position of the drill guide sleeve relative to the surface of the longitudinally extending side wall of a bone where the frame is attached at one end thereof. For example, when the frame is attached to the spherical head part of a long bone such as a femur, approximately on the axis of the femoral neck, the second limb of the stylus can trace around the femoral neck or the base of the spherical head, to assess the transactional alignment of the drill guide sleeve, or its angular alignment, or both.

Preferably; the length of at least one of the first and second limbs of the stylus is adjustable. For example, one or each of the limbs can comprise first and second telescoping parts. The length of the or each adjustable limb should be capable of being locked, for example by means of a threaded fastener.

Preferably, the frame has three legs by which it can be fitted on to a bone. This can enable the frame to be fixed to the bone in a stable configuration. Each of the legs can have a hole at its lower end for receiving a fastener by which it can be fixed to the bone, for example by means of a bone screw or a pin.

The instrument will generally be made from metals which are conventionally used in the manufacture of surgical instruments. Certain stainless steels are particularly preferred.

Embodiments of the present invention will now be described by way of example with reference to the accompanying drawings, in which:
Figure 1 is a side view of the head of a femur, showing part of a drill guide assembly according to the invention mounted on the femur head.
Figure 2 is a side view of a drill guide for use in an assembly as shown in Figure 1.
Figure 3 is a side view of a drill guide assembly which can be attached to a femur in the way shown in Figure 1.
Figure 4 is cross-sectional elevation through a part of the assembly shown in Figure 3.

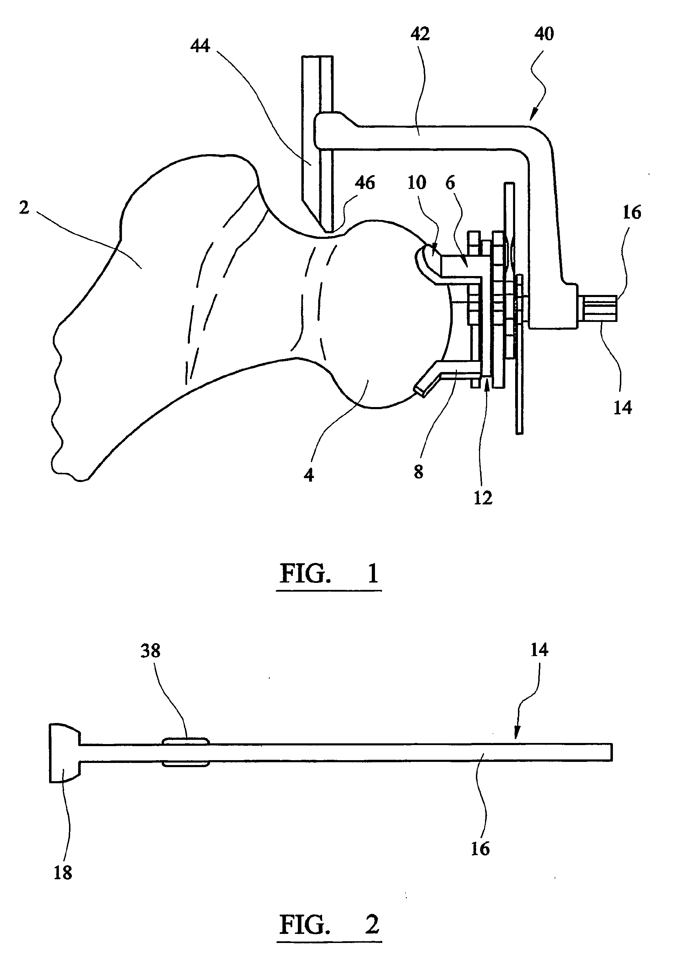

Referring to the drawings, Figure 1 shows the upper portion of a femur 2, including the head 4 which is received in the patient's acetabulum during articulation of the joint. The femur is shown in isolation to show how the present invention can be put into effect to prepare the femur for implantation of a "resurfacing" joint prosthesis component. This can be used to provide a hard wearing bearing surface on the head of the femur, which can articulate with an appropriate prosthesis component which is implanted in the acetabulum. The use of such surgical techniques has the advantage that most of the femoral tissue is preserved.

The preparation of the head of the femur to receive a resurfacing prosthesis component involves the formation of a bore along the axis of the head, to receive the stem of the component. The external surface of the head is prepared by the formation of two rotationally symmetrical reamed surfaces. A first surface is aligned parallel or at a predetermined angle relative to the axis of the head. A second surface extends between the first surface and the axis, the angle between the first and second surfaces being about 135°. The preparation of the external surface of the head requires determination of the axis for the component.

Figure 2 shows a drill guide 14 which comprises a sleeve 16 in which the size of the bore is slightly bigger than the size of a drill which is to extend through the sleeve to create a bore in the patient's bone. The sleeve has an enlarged bulb 18 at its lower end. Portions of the side walls of the bulb are generally spherical. The sleeve 16 has a thread 38 formed in its side wall along part of its length.

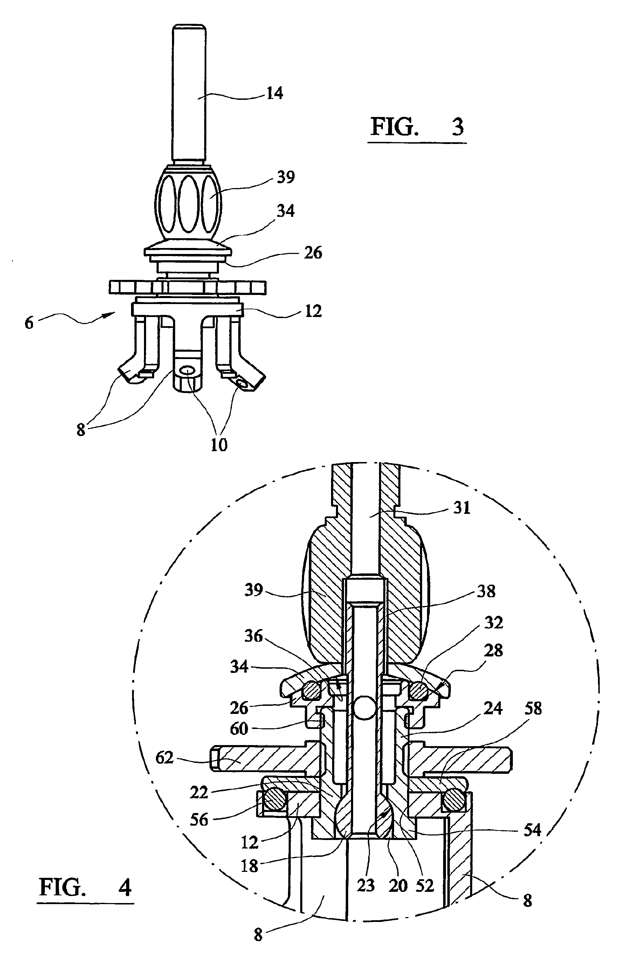

Figures 3 and 4 show the assembly of the invention which comprises the drill guide shown in Figure 2 and a frame 6. The frame which has three legs 8 for mounting onto the bone surface. A hole 10 extends through each of the legs at its base, for receiving a fixing screw. The legs are equally spaced around a platform 12. The platform comprises a circular ring, from which the legs depend.

The bulb 18 on the sleeve 14 is received in a recess 20 in a housing 22. The internal surface 23 of the recess which faces towards the bone has a generally rounded shape.

The housing includes an upstanding sheath 24 which extends from the recess 20 in a direction away from the surface of the bone to which the assembly is attached. A collar 26 is provided on the sheath, which provides a clamping surface 28 facing away from the surface of the bone. The clamping surface has a groove formed in it, with an elastomeric O-ring 32 in the groove. The clamping surface on the collar has a convex domed shape. In the illustrated embodiment, the sheath 24 is formed integrally with that part of the housing which defines the recess 20, and the collar is provided by a separate part which is fastened to the housing by means of screw threads.

The sheath 24 has a bore 31 through it, which communicates with an opening in the recess 20, so that the drill guide sleeve can extend upwardly from the recess, through the bore 31 in the sheath 24, and through an opening in the collar 26.

The drill guide sleeve has a washer 34 positioned on it which provides a clamping surface 36 facing towards the clamping surface 28 on the collar 26. The washer is a sliding fit over the drill guide sleeve. The clamping surface on the washer has a concave domed shape.

The drill guide sleeve has a thread 38 formed in its external surface. A nut 39 with a mating internal thread is threaded on to the external surface of the drill guide sleeve.

The openings in the recess 20 and in the collar 26, and the bore 31 in the sheath 24 are sized to allow the angular orientation of the drill guide sleeve 14 relative to the frame 6 to be adjusted, for example through an angle of up to about 20° or more. Such movement of the drill guide sleeve is possible when the nut 39 is unscrewed on the threaded external surface of the drill guide sleeve, so that the clamping surface 36 on the underside of the washer 34 is able to move easily relative to the O-ring 32 and the clamping surface 28 on the collar 26. The nut 39 can be used to lock the drill guide sleeve against angular adjustment.

The nut 39 can be tightened down on to the washer 34, to urge the washer against the O-ring and the collar. This tends to draw the bulb 18 against the rounded internal surface 23 of the recess 20 in the housing 22. The action of the internal surface of the housing against the bulb, and of the O-ring or collar or both against the washer, prevent relative movement between the housing and the bulb and between the collar and the washer. The tight sliding fit between the washer and the drill guide sleeve prevent relative movement between the collar and the drill guide sleeve.

Locking the drill guide against movement relative to the housing in the embodiment shown in Figure 4 involves drawing the drill guide bulb upwardly against the clamping surface provided by the recess in the housing, and drawing the washer downwardly against the collar. A reverse arrangement can be used, in which the drill guide bulb is forced downwardly against an upwardly facing clamping surface provided by the recess in the housing, with appropriate rearrangement of the upper clamping surfaces.

Figure 4 also shows details of the lock mechanism by which the drill guide can be locked against translation across the frame. The platform 12 from which each of the legs 6 depend has an opening 52 in it in which the housing 22 is located. The opening 52 is larger than the housing, allowing the housing to translate across the platform: as shown in Figure 3, translation occurs in a direction which is perpendicular to the plane of the drawing.

The housing has a lug 54 around its lower edge which is larger than the transverse dimension of the opening 52 in the platform 12. The lug prevents the housing from being pulled through the opening, in a direction away from the bone.

The platform 12 has a groove formed in its upper surface, which contains an elastomeric O-ring 56. A rigid washer 58 is positioned on the housing, in face-to-face relationship with the upper surface of the platform and in contact with the O-ring 56.

The external surface of the housing has a thread 60 formed in it. A threaded lock nut 62 engages the thread 60 on the housing, and can act against the washer 58 when it is tightened. Such action against the washer tends to draw the housing upwardly relative to the platform. The platform is then clamped between the lug 54 and the washer 58, preventing movement of the housing (with the drill guide located within it) relative to the frame.

As shown in Figure 1, the assembly of the invention includes a stylus 40 which can be rotated about the axis which is defined by the sleeve 16 of the drill guide. The stylus comprises a first limb 42 which extends in a direction generally away from the platform towards the bone, and a second limb 44 extending from the first limb in a direction generally towards the axis of the assembly, the second limb having a stylus tip 46. The effective length of the second limb can be adjusted by means of a sliding engagement of the second limb in a track on the first limb.

The assembly of the invention can be used to locate the axis on which the spherical head of a femur or similar long bone is prepared for resection prior to implantation of a joint prosthesis component. In a first step, the axis of the bone is determined approximately, for example by eye, or using instruments, with reference to other anatomical features of the patient's bone. Instruments which can be used for this purpose in relation to a patient's femur are disclosed in WO-A-03/026517 and WO-A-03/026518. The frame is then attached relative to this axis by means of fasteners which pass through the holes 10 in the bases of the legs 8 on the platform, into the bone tissue.

The locks which determine the freedom of movement of the drill guide relative to the platform can be released to allow one or both of angular adjustment and translation. The stylus can be rotated about the axis of the bone to determine the position of the drill guide relative to the surface of the bone which can be traced out using the stylus. When the stylus can be rotated about the spherical head of the bone with a constant distance between the stylus tip and the bone surface, the drill guide will be aligned with the axis of the head. This will generally be the desired location for drilling the bone. However, in some applications, it can be desired for the drill guide to be located off the bone axis.

## Claims

1. A drill guide assembly for determining the axis for drilling a bore to receive a component of an orthopaedic joint prosthesis, which comprises:
a. a drill guide (14) which comprises a sleeve (16) and a bulb (18) at one end of the sleeve,
b. a frame (6) which can be fastened to a bone, including a housing (22) which defines a recess (20) in which the drill guide bulb can be received with the drill guide sleeve extending out of the recess, in a direction away from the bone, so that the angular orientation of the drill guide sleeve relative to the housing can be adjusted by movement of the bulb within the recess, and
c. a clamp for locking the drill guide relative to the housing against angular adjustment,
in which the clamp comprises a lower pair of clamping surfaces provided by the drill guide bulb and the internal wall of the recess respectively, and an upper pair of clamping surfaces on the drill guide and the housing respectively, **characterised in that** the lower pair of clamping surfaces and the upper pair of clamping surfaces are arranged so that the drill guide can be locked against angular adjustment by engagement between the frame clamping surfaces and the drill guide clamping surfaces of each of the lower and upper pairs, in which the upper clamping surface of the drill guide is spaced apart from the bulb along the drill guide sleeve.

2. A drill guide assembly as claimed in claim 1, in which the frame upper clamping surface is provided on a collar (26) which extends from the housing (22) in a direction away from the bone, the collar being hollow so that the drill guide sleeve (16) can extend through it.

3. A drill guide assembly as claimed in claim 1, in which the frame upper clamping surface faces generally away from the bone and the clamping surface of the recess (20) faces generally towards the bone.

4. A drill guide assembly as claimed in claim 1, in which one of the upper clamping surfaces is provided by a washer (34), and the clamp includes an actuator (39) which can act on the washer to urge it against the other of the upper clamping surfaces.

5. A drill guide assembly as claimed in claim 4, in which the actuator comprises a threaded nut.

6. A drill guide assembly as claimed in claim 1, which includes a resiliently deformable washer which is located between at least one of the upper clamping surfaces and the lower clamping surfaces.

7. A drill guide assembly as claimed in claim 1, in which the ratio of (a) the distance between the upper and lower clamping surfaces when the drill guide is clamped against angular adjustment to (b) the transverse dimension of the bulb, measured perpendicular to the axis of the drill guide sleeve, is at least 1.3.

8. A drill guide assembly as claimed in claim 1, in which the frame (6) provides a platform (12) which defines a plane which is spaced apart from the bone and an axis of the assembly which extends perpendicular to the said plane, and in which the drill guide is mounted on the platform so that it can be translated relative to the frame generally in the plane of the platform.

9. A drill guide assembly as claimed in claim 8, which includes a lock (62) for preventing translation of the drill guide relative to the frame, in which the clamp and the lock can be engaged and disengaged independently of one another.

10. A drill guide assembly as claimed in claim 1, which includes an alignment stylus (40) connected to the drill guide to move with the drill guide relative to the frame, the stylus including a first limb (42) which is directed towards the bone, to facilitate assessment of the alignment of the drill guide sleeve relative to anatomical features of the bone.

11. A drill guide assembly as claimed in claim 10, in which the stylus can be moved rotatably around the drill guide sleeve.

12. A drill guide assembly as claimed in claim 11, in which the stylus can be moved around the drill guide sleeve while the clamp is engaged to prevent angular movement of the drill guide relative to the frame.

13. A drill guide assembly as claimed in claim 10, in which the stylus includes a second limb (44) exending from the first limb in a direction generally towards the axis of the assembly.

14. A drill guide assembly as claimed in claim 13, in which the length of at least one of the first and second limbs of the stylus is adjustable.

15. A drill guide assembly as claimed in claim 1, in which the frame has three legs (8) by which it can be fitted on to a bone.

## Patentansprüche

1. Bohrlehre zur Festlegung der Achse zum Bohren einer Bohrung für die Aufnahme einer Komponente einer orthopädischen Gelenkprothese, die aufweist:
a. eine Bohrerführung (14), die eine Hülse (16) und einen Wulst (18) an einem Ende der Hülse aufweist,
b. einen Rahmen (6), der an einem Knochen befestigbar ist und ein Gehäuse (22) enthält, das eine Aussparung (20) definiert, in der der Wulst der Bohrerführung bei sich aus der Aussparung, in einer Richtung vom Knochen weg, erstreckender Hülse der Bohrführung aufnehmbar ist, sodass die Winkelorientierung der Hülse der Bohrerführung relativ zum Gehäuse durch Bewegung des Wulstes in der Aussparung einstellbar ist, und
c. eine Klemme zum Feststellen der Bohrerführung relativ zum Gehäuse gegen Winkelverstellung,
in der die Klemme ein unteres Paar von Klemmflächen, die von dem Wulst der Bohrerführung beziehungsweise der Innenwand der Aussparung bereitgestellt werden, und ein oberes Paar von Klemmflächen an der Bohrerführung beziehungsweise dem Gehäuse aufweist, **dadurch gekennzeichnet, dass** das untere Paar von Klemmflächen und das obere Paar von Klemmflächen so angeordnet sind, dass die Bohrerführung gegen Winkelverstellung durch Eingriff zwischen den Rahmenklemmflächen und den Bohrerführungsklemmflächen von jedem der unteren und oberen Paare feststellbar ist, wobei die obere Klemmfläche der Bohrerführung von dem Wulst entlang der Hülse der Bohrerführung beabstandet ist.

2. Bohrlehre nach Anspruch 1, **dadurch gekennzeichnet, dass** die obere Klemmfläche des Rahmens an einem Kragen (26) vorgesehen ist, der sich von dem Gehäuse (22) in eine Richtung vom Knochen weg erstreckt, wobei der Kragen hohl ist, sodass sich die Hülse (16) der Bohrerführung durch ihn hindurch erstrecken kann.

3. Bohrlehre nach Anspruch 1, **dadurch gekennzeichnet, dass** die obere Klemmfläche des Rahmens allgemein vom Knochen weg zeigt und die Klemmfläche der Aussparung (20) allgemein in Richtung zum Knochen zeigt.

4. Bohrerführung nach Anspruch 1, **dadurch gekennzeichnet, dass** eine der oberen Klemmflächen durch eine Unterlegscheibe (34) bereitgestellt ist, und dass die Klemme einen Aktuator (39) enthält, der auf die Unterlegscheibe einwirken kann, um sie gegen die andere der oberen Klemmflächen zu zwingen.

5. Bohrlehre nach Anspruch 4, **dadurch gekennzeichnet**, das der Aktuator eine Gewindemutter aufweist.

6. Bohrlehre nach Anspruch 1, **dadurch gekennzeichnet, dass** sie eine elastisch verformbare Unterlegscheibe enthält, die sich zwischen den oberen Klemmflächen und/oder unteren Klemmflächen befindet.

7. Bohrlehre nach Anspruch 1, **dadurch gekennzeichnet, dass** das Verhältnis (a) der Distanz zwischen den oberen und unteren Klemmflächen, wenn die Bohrerführung gegen Winkelverstellung geklemmt ist, (b) zur Querabmessung des Wulstes, senkrecht zur Achse der Hülse der Bohrerführung gemessen, mindestens 1,3 beträgt.

8. Bohrlehre nach Anspruch 1, **dadurch gekennzeichnet, dass** der Rahmen (6) eine Plattform (12), die eine Ebene definiert, die vom Knochen beabstandet ist, und eine Achse der Anordnung bereitstellt, die sich senkrecht zur Ebene erstreckt, und dass die Bohrerführung auf der Plattform so montiert ist, dass sie relativ zum Rahmen allgemein in der Ebene der Plattform verschoben werden kann.

9. Bohrlehre nach Anspruch 8, **dadurch gekennzeichnet, dass** sie eine Verriegelung (62) zum Verhindern einer Translation der Bohrerführung relativ zum Rahmen enthält, und dass die Klemme und die Verriegelung unabhängig voneinander in Eingriff gebracht und außer Eingriff gebracht werden können.

10. Bohrlehre nach Anspruch 1, **dadurch gekennzeichnet, dass** sie einen Ausrichtestift (40) enthält, der zum Bewegen mit der Bohrerführung relativ zum Rahmen mit der Bohrerführung verbunden ist, wobei der Stift eine ersten Schenkel (42) enthält, der in Richtung zum Knochen gerichtet ist, um eine Beurteilung der Ausrichtung der Hülse der Bohrerführung relativ zu anatomischen Merkmalen des Knochens zu erleichtern.

11. Bohrlehre nach Anspruch 10, **dadurch gekennzeichnet, dass** der Stift um die Hülse der Bohrerführung drehbar bewegbar ist.

12. Bohrlehre nach Anspruch 11, **dadurch gekennzeichnet, dass** der Stift um die Hülse der Bohrerführung bewegbar ist, während die Klemme im Eingriff steht, um eine Winkelbewegung der Bohrerführung relativ zum Rahmen zu verhindern.

13. Bohrlehre nach Anspruch 10, **dadurch gekennzeichnet, dass** der Stift einen zweiten Schenkel (44) enthält, der sich von dem ersten Schenkel in einer Richtung allgemein in Richtung zur Achse der Anordnung erstreckt.

14. Bohrlehre nach Anspruch 13, **dadurch gekennzeichnet, dass** die Länge von dem ersten und/oder zweiten Schenkel des Stiftes verstellbar ist.

15. Bohrlehre nach Anspruch 1, **dadurch gekennzeichnet, dass** der Rahmen drei Beine (8) aufweist, durch die er an einen Knochen angepasst werden kann.

## Revendications

1. Ensemble guide-foret pour déterminer l'axe de perçage d'un alésage pour recevoir un composant d'une prothèse d'articulation orthopédique, comprenant :
a. un guide-foret (14) comprenant un manchon (16) et une ampoule (18) à une extrémité du manchon,
b. une armature (6) qui peut être fixée à un os, comprenant un logement (22) définissant un évidement (20) dans lequel l'ampoule du guide-foret peut être logée, le manchon du guide-foret s'étendant hors de l'évidement, dans une direction opposée à l'os, de telle sorte que l'orientation angulaire du manchon du guide-foret par rapport au logement puisse être ajustée par le mouvement de l'ampoule à l'intérieur de l'évidement, et
c. un élément de serrage pour verrouiller le guide-foret par rapport au logement contre l'ajustement angulaire,
dans lequel l'élément de serrage comprend une paire inférieure de surfaces de serrage fournies par l'ampoule du guide-foret et la paroi intérieure de l'évidement, respectivement, et une paire supérieure de surfaces de serrage sur le guide-foret et le logement, respectivement, **caractérisé en ce que** la paire inférieure de surfaces de serrage et la paire supérieure de surfaces de serrage sont disposées de telle sorte que le guide-foret puisse être verrouillé contre l'ajustement angulaire par l'accouplement entre les surfaces de serrage de l'armature et les surfaces de serrage du guide-foret de chacune des paires inférieure et supérieure, la surface de serrage supérieure du guide-foret étant espacée de l'ampoule le long du manchon du guide-foret.

2. Ensemble de guide-foret selon la revendication 1, dans lequel la surface de serrage supérieure de l'armature est disposée sur un collier (26) s'étendant depuis le logement (22) dans une direction opposée à l'os, le collier étant creux, de telle sorte que le manchon du guide-foret (16) puisse s'étendre à travers celui-ci.

3. Ensemble de guide-foret selon la revendication 1, dans lequel la surface de serrage supérieure de l'armature est orientée globalement dans une direction opposée à l'os et la surface de serrage de l'évidement (20) est orientée globalement vers l'os.

4. Ensemble de guide-foret selon la revendication 1, dans lequel l'une des surfaces de serrage supérieures est fournie par une rondelle (34), et l'élément de serrage comprend un actionneur (39) qui peut agir sur la rondelle pour la pousser contre l'autre des surfaces de serrage supérieures.

5. Ensemble de guide-foret selon la revendication 4, dans lequel l'actionneur comprend un écrou fileté.

6. Ensemble de guide-foret selon la revendication 1, comprenant une rondelle déformable résiliente située entre au moins l'une des surfaces de serrage supérieures et des surfaces de serrage inférieures.

7. Ensemble de guide-foret selon la revendication 1, dans lequel le rapport de (a) la distance entre les surfaces de serrage supérieures et inférieures quand le guide-foret est serré contre l'ajustement angulaire sur (b) la dimension transversale de l'ampoule, mesuré perpendiculairement à l'axe du manchon du guide-foret, est d'au moins 1,3.

8. Ensemble de guide-foret selon la revendication 1, dans lequel l'armature (6) fournit une plate-forme (12) qui définit un plan espacé de l'os et un axe de l'ensemble qui s'étend perpendiculairement audit plan, et dans lequel le guide-foret est monté sur la plate-forme de telle sorte qu'il puisse être déplacé en translation par rapport à l'armature globalement dans le plan de la plate-forme.

9. Ensemble de guide-foret selon la revendication 8, comprenant un verrou (62) pour empêcher la translation du guide-foret par rapport à l'armature, dans lequel l'élément de serrage et le verrou peuvent être accouplés et désaccouplés indépendamment l'un de l'autre.

10. Ensemble de guide-foret selon la revendication 1, comprenant un stylet d'alignement (40) relié au guide-foret pour se déplacer avec le guide-foret par rapport à l'armature, le stylet comprenant un premier membre (42) dirigé vers l'os, pour faciliter l'évaluation de l'alignement du guide-foret par rapport aux caractéristiques anatomiques de l'os.

11. Ensemble de guide-foret selon la revendication 10, dans lequel le stylet peut être déplacé de façon rotative autour du manchon du guide-foret.

12. Ensemble de guide-foret selon la revendication 11, dans lequel le stylet peut être déplacé autour du manchon du guide-foret alors que l'élément de serrage est accouplé pour empêcher le mouvement angulaire du guide-foret par rapport à l'armature.

13. Ensemble de guide-foret selon la revendication 10, dans lequel le stylet comprend un second membre (44) s'étendant depuis le premier membre dans une direction globalement vers l'axe de l'ensemble.

14. Ensemble de guide-foret selon la revendication 13, dans lequel la longueur d'au moins un membre parmi les premier et second membre du stylet est ajustable.

15. Ensemble de guide-foret selon la revendication 1, dans lequel l'armature comporte trois pieds (8) grâce auxquels elle peut être fixée sur un os.
